# EUROPÄISCHE PATENTSCHRIFT

(11) **EP 0 695 162 B1**
(45) Veröffentlichungstag und Bekanntmachung des Hinweises auf die Patenterteilung: **18.06.1997**
(21) Anmeldenummer: 94915084.1
(22) Anmeldetag: 21.04.1994
(51) Int. Cl.: A61K 7/13

(54) **ISATINHALTIGE MITTEL ZUM FÄRBEN VON KERATINHALTIGEN FASERN**
DYES CONTAINING ISATIN FOR KERATIN-CONTAINING FIBRES
AGENTS CONTENANT DES ISATINES PERMETTANT DE COLORER DES FIBRES CONTENANT DE LA KERATINE

(30) Priorität: 30.04.1993 DE 4314317
(43) Veröffentlichungstag der Anmeldung: 07.02.1996
(73) Patentinhaber: Henkel Kommanditgesellschaft auf Aktien, 40191 Düsseldorf (DE)
(72) Erfinder: MÖLLER, Hinrich, D-40789 Monheim (DE); HÖFFKES, Horst, D-40595 Düsseldorf (DE)
(86) Internationale Anmeldenummer: EP9401246
(87) Internationale Veröffentlichungsnummer: WO9424988

(56) Entgegenhaltungen:
- EP-A- 0 359 465
- EP-A- 0 497 697
- EP-A- 0 502 783
- EP-A- 0 502 784
- DE-A- 2 716 671

## Beschreibung

Gegenstand der Erfindung sind Mittel zum Färben von keratinhaltigen Fasern, insbesondere menschlichen Haaren, die Isatine und eine weitere Verbindung enthalten. In einer bevorzugten Ausführungsform enthalten diese Mittel zusätzlich ein Ammonium- oder Metallsalz.

Fur das Farben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von Oxidationsmitteln wie z. B. H₂O₂, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbesysteme auf Basis von Isatin oder Isatinderivaten bieten hier eine Alternative. Isatin ist als Direktfarbstoff zum Färben von Keratinfasern alleine oder in Kombination mit Chinonfarbstoffen in der deutschen Offenlegungsschrift DE 36 35 147 A1 beschrieben worden.
Die Variationsbreite der erzielbaren Nuancen ist jedoch beschränkt.
In den allermeisten Fällen erhält man eine goldfarbene Färbung.

Ein weiteres isatinhaltiges Färbesystem wird in der europäischen Offenlegungsschrift EP 359 465 A2 beschrieben. Hier wird die Färbung mit Hilfe eines aus der Reaktion eines Isatins mit einem Anilinderivat entstehenden Ketimins (Schiff'sche Base) erzielt. Das Ketimin wird entweder als solches auf keratinische Fasern aufgebracht und entwickelt dort eine Färbung, oder aber eine aus einem Isatin und einem Anilinderivat bestehende Mischung wird auf die Faser aufgebracht und bildet zunächst "in situ" das Ketimin, woraufhin sich auf der Faser die Färbung entwickelt.

Die europäische Offenlegungsschrift EP 497 697 A1 beschreibt Haarfärbemittel auf Basis von Isatinen und Aminoindolen oder -indolinen mit primärer Aminogruppe, wobei sich in einer Kondensationsreaktion Schiff'sche Basen bilden.

Die europäische Offenlegungsschrift EP 0 502 783 A1 beschreibt Haarfärbemittel, die Isatine und Aminopyridine oder Isatine und Aminopyrimidine mit primärer Aminogruppe enthalten.

Die europäische Offenlegungsschrift EP 0 502 784 A1 beschreibt Haarfärbemittel, die Isatine und substituierte Diamine oder Aminophenole oder aber Isatine und (Bisaryl)-alkylendiamine enthalten.

Überraschenderweise wurde nun gefunden, daß sich Isatine in Kombination mit einer Reihe von weiteren Verbindungen zum Färben von keratinhaltigen Fasern eignen, darunter auch sekundäre und tertiäre Amine, also Amine, die nicht in der Lage sind, Schiff'sche Basen zu bilden.

Als keratinhaltige Fasern kommen z. B. Wolle, Pelze, Felle und menschliche Haare in Betracht. Obwohl die besten Färbungen an Keratinfasern erzielt werden, können die erfindungsgemäßen Färbemittel auch prinzipiell zum Färben anderer Naturfasern wie z. B. Baumwolle, Jute, Sisal, Leinen oder Seide, modifizierter Naturfasern wie z.B. Regeneratcellulose, Nitro-, Alkyl-, Hydroxyalkyl- oder Acetylcellulose und synthetischer Fasern wie z. B. Polyamid-, Polyacrylnitril-, Polyurethan- und Polyesterfasern verwendet werden.

Die im folgenden näher bezeichneten Färbemittel verfügen über sehr gute anwendungstechnische Eigenschaften wie z. B. Egalisiervermögen und Farbaufzugvermögen. Die mit den erzielten Färbemitteln erzielten Färbungen sind lichtecht, reibecht, waschecht und verfügen über eine gute Beständigkeit gegenüber Dauerwellflüssigkeiten.

Gegenstand der Erfindung sind Mittel zum Färben von keratinhaltigen Fasern enthaltend

Mittel zum Färben von keratinhaltigen Fasern enthaltend
- mindestens ein Isatinderivat der Formel I 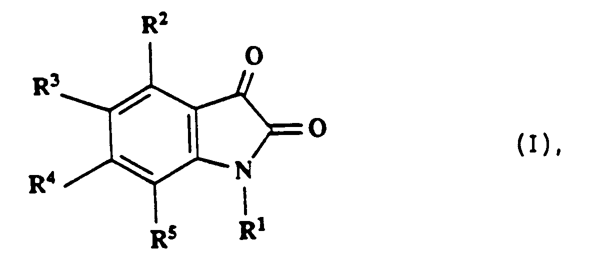 wobei R¹ Wasserstoff, eine C₁-C₄-Alkylgruppe, eine C₂-C₄-Hydroxyalkylgruppe, eine C₂-C₂₀-Acylgruppe, eine Phenylgruppe oder eine Benzoylgruppe und R², R³, R⁴ und R⁵ unabhängig voneinander Wasserstoff, Hydroxy, Halogen, Nitrogruppen, Sulfogruppen, Carboxylgruppen, C₁-C₄-Alkylgruppen, C₁-C₄-Alkoxygruppen oder NR⁶R⁷-Gruppen bedeuten, wobei R⁶ und R⁷ unabhängig voneinander Wasserstoff, C₁-C₄-Alkylgruppen oder C₂-C₄-Hydroxyalkylgruppen darstellen, und zwei benachbarte Gruppen R³, R⁴ und R⁵ auch eine Alkylendioxygruppe mit 1 bis 4 C-Atomen darstellen können,
- mindestens eine Verbindung ausgewählt aus der Gruppe
   a) der primären aliphatischen Amine, die in der C-Kette mindestens eine zusätzliche Gruppe NR⁸R⁹ oder OR¹⁰ tragen, wobei R⁸, R⁹ und R¹⁰ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten,
   b) der heterocyclischen oder isocyclischen aromatischen Verbindungen ohne primäre Aminogruppe,
   c) der aromatischen Carbon- und Sulfonsäuren mit primärer Aminogruppe,
   d) der Anilinderivate der Formel II 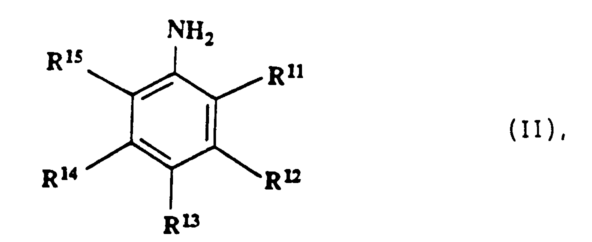 wobei R¹¹, R¹², R¹³, R¹⁴ und R¹⁵ Wasserstoff, C₁-C₄-Alkylgruppen, C₂-C₄-Hydroxyalkylgruppen, C₂-C₄-(C₁-C₄-Alkoxy)-alkylgruppen oder Gruppen NR¹⁶R¹⁷ oder OR¹⁸ darstellen, wobei R¹⁶, R¹⁷ und R¹⁸ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten, mit der Maßgabe, daß höchstens zwei der Gruppen R¹¹ bis R¹⁵ keine NR¹⁶R¹⁷- und/oder OR¹⁸-Gruppe darstellen,
   e) der Anilinderivate der Formel III 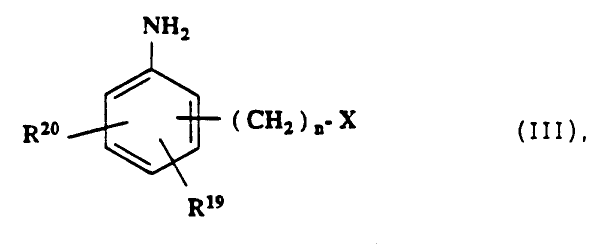 wobei n für eine ganze Zahl von 1 bis 4 und X für eine Hydroxy- oder Aminogruppe steht und R¹⁹ und R²⁰ Wasserstoff, C₁-C₄-Alkylgruppen, C₂-C₄-Hydroxyalkylgruppen, C₂-C₄-(C₁-C₄-Alkoxy)-alkylgruppen oder Gruppen NR²¹R²² oder OR²³ darstellen, wobei R²¹, R²² und R²³ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten, mit der Maßgabe, daß mindestens eine der Gruppen R¹⁹ und R²⁰ eine Gruppe NR²¹R²² oder OR²³ darstellt,
   f) der Dianilinderivate der Formel IV 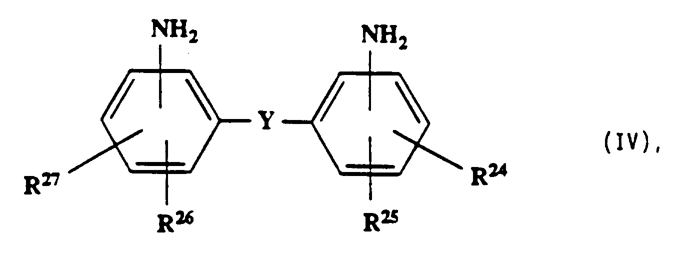 wobei Y für eine direkte Bindung oder für eine Gruppe CO, SO, O, S oder O-(CH₂-Z-CH₂-O)ₘ steht, wobei Z für eine direkte Bindung, eine Gruppe CH₂, CHOH oder CH₂OC₂H₄OCH₂ steht, und m eine ganze Zahl von 1 bis 4 bedeutet, oder Y auch eine gesättigte oder ungesättigte Alkylengruppe mit 1 bis 4 C-Atomen, die gegebenenfalls durch OH substituiert sein kann, bedeutet und R²⁴, R²⁵, R²⁶ und R²⁷ Wasserstoff, C₁-C₄-Alkylgruppen, C₂-C₄-Hydroxyalkylgruppen, C₂-C₄-(C₁-C₄-Alkoxy)-alkylgruppen oder Gruppen NR²⁸R²⁹ oder OR³⁰ darstellen, wobei R²⁸, R²⁹ und R³⁰ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten, mit der Maßgabe, daß jeweils mindestens eine der Gruppen R²⁴ und R²⁵ und eine der Gruppen R²⁶ und R²⁷ eine Gruppe NR²⁸R²⁹ oder OR³⁰ darstellt,
   g) der nicht-aromatischen unsubstituierten oder mit einer der Gruppen Amino-(C₁-C₄)-alkyl, Hydroxy-(C₁-C₄)-alkyl oder Carboxyl substituierten Heterocyclen,
   h) der Aminozucker
- und einen wasserhaltigen Träger.

Besonders gute Färbeergebnisse erzielt man mit den erfindungsgemäßen Mitteln, wenn im Isatinderivat der Formel I R¹ Wasserstoff ist und R², R³, R⁴ und R⁵ unabhängig voneinander Wasserstoff, Hydroxy, Halogen, Methyl-, Sulfogruppen oder NR⁶R⁷-Gruppen darstellen, worin R⁶ und R⁷ Wasserstoff bedeuten. Besonders bevorzugt ist der Grundkörper Isatin selbst.

In allen Fällen können auch die Salze der Isatinderivate der Formel I und der unter a) bis g) aufgeführten Verbindungen verwendet werden. Im Falle von Verbindungen mit Aminfunktionen kommen z. B. die Sulfate, Hydrochloride oder Hydrobromide in Frage, im Falle von Verbindungen mit Carboxy- oder Sulfofunktionen kommen z. B. die Alkali- oder Ammoniumsalze in Frage oder aber auch innere Salze.

Es könne auch Gemische von verschiedenen Isatinen der Formel I oder deren Salzen verwendet werden. Außerdem können auch Gemische der unter a) bis g) aufgeführten Verbindungen verwendet werden.

Bevorzugte Mittel zum Färben von keratinhaltigen Fasern sind solche, in denen
- die oben unter a) näher bezeichneten
   aliphatischen Amine ausgewählt sind aus 2-Aminoethanol, 2-Methoxyamin, 2-Ethoxyethylamin, 2-(2-Aminoethoxy)-ethanol, 2-Aminopropanol, 3-Aminopropanol, 2,3-Dihydroxypropylamin, 4-Hydroxypropylamin, 2-Aminopropan-1,3-diol, 2-Amino-2-methylpropanol, 2-Amino-2-methylpropan-1,3-diol, 2-Amino-2-hydroxymethylpropan-1,3-diol, Tetrahydroxypentylaminen, Pentahydroxyhexylaminen, 1,2-Diaminoethan, 1,2-Diaminopropan, 1,3-Diaminopropan, 1,3-Diamino-2-propanol, 2-(2-Aminoethylamino)-ethylamin, 2-(2-Aminoethylamino)-ethanol, 3-(2-Aminoethylamino)-propylamin, 3-(2-Aminoethylamino)-propanol,
- die oben unter b) aufgeführten heterocyclischen oder isocyclischen aromatischen Verbindungen ohne primäre Aminogruppe ausgewählt sind aus Indolin, Indol, Pyrrol, 3-Pyrrolin, Pyrrolidin, 1-Methylpyrrol, 2-Methylpyrrol, 3-Methylpyrrol, 2,5-Dimethylpyrrol, Pyrazol, 3-Methylpyrazol, Imidazol, Indoxylacetat, Tetrahydrochinolin, Tetrahydroisochinolin, 2-Indolcarbonsäure, 3-Indolylessigsäure, 4-Dimethylaminopyridin, 2,6-Dihydroxy-3,4-dimethylpyridin, Pyrrol-2-carbonsäure, 2-Methylresorcin,
- die oben unter c) aufgeführten aromatischen Carbon- und Sulfonsäuren mit primärer Aminogruppe ausgewählt sind aus 2-, 3- oder 4-Aminobenzoesäure, 2-, 3- oder 4-Phenylessigsäure, 2,3-, 2,4-, 2,5-, 3,4- oder 3,5-Diaminobenzoesäure, 4- oder 5-Aminosalicylsäure, 3-Amino-4-hydroxybenzoesäure, 4-Amino-3-hydroxybenzoesäure, 2-, 3- oder 4-Aminobenzolsulfonsäure, 4-Amino-3-hydroxynaphthalin-1-sulfonsäure, 3-Aminophtalsäure, 5-Aminoisophtalsäure,
- die oben unter d) näher bezeichneten Anilinderivate der Formel II ausgewählt sind aus 1,2,4,5-Tetraaminobenzol, 2,4,5-Triaminophenol, Pentaminobenzol, Hexaaminobenzol, 2,4,6-Triaminoresorcin, 4,5-Diaminobrenzkatechin, 4,6-Diaminopyrogallol, 2,5-Dihydroxy-4-morpholinoanilin 3,5-Diamino-4-hydroxybrenzkatechin,
- die oben unter e) näher bezeichneten Anilinderivate der Formel III ausgewählt sind aus 2-(2,5-Diaminophenyl)-ethanol, 3-Aminomethyl-4-aminophenol, 3-(2,5-Diaminophenyl)-propanol, 2-Aminomethyl-4-aminophenol, 2-Aminomethyl-5-aminophenol, 2-(2,4-diaminophenyl)-ethanol,
- die oben unter f) näher bezeichneten Dianilinderivate der Formel VI ausgewählt sind aus 4,4'-Diaminostilben, 4,4'-Diaminostilben-2,2'-disulfonsäure, 4,4'-Diaminodiphenylmethan, 4,4'-Diaminodiphenylsulfid, 4,4'-Diaminodiphenylsulfoxid, 4,4'-Diaminobenzophenon, 4,4'Diaminobenzodiphenylether, 3,3',4,4'-Tetraaminodiphenyl, 3,3',4,4'-Tetraaminobenzophenon, 1,3-Bis-(2,4-diaminophenoxy)-propan, 1,8-Bis-(2,5-diaminophenoxy)-3,6-dioxaoctan,
- die oben unter g) aufgeführten nicht-aromatischen Heterocyclen ausgewählt sind aus Piperidin, Piperazin, 1-(2-Aminooctyl)-piperazin, 1-(2-Hydroxyethyl)-piperazin, 3-Pyrrolin, Pyrrolidin, Thiazolidin, Thiazolidin-4-carbonsäure, Piperidin-2-carbonsäure, Piperidin-3-carbonsäure, Piperidin-4-carbonsäure.
- die oben unter h) aufgeführten Aminozucker ausgewählt sind aus D-Glucosamin und D-Galactosamin.

Wenn man den erfindungsgemäßen Färbemitteln bestimmte Ammonium- oder Metallsalze zusetzt, erhält man bei relativ niedrigen Temperaturen bereits nach kurzer Zeit besonders farbintensive Ausfärbungen.

Ein weiterer Erfindungsgegenstand sind deshalb Mittel zum Färben von keratinhaltigen Fasern, die mindestens ein Salz ausgewählt aus der Gruppe der Ammonium-, Lithium-, Natrium-, Kalium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze enthalten.

Besonders geeignete Salze sind Ammoniumcarbonat, Ammoniumacetat, Natriumacetat, Lithiumacetat, Kaliumacetat, Natriumglykolat, Natriumlactat, Calciumgluconat.Die erfindungsgemäßen Färbemittel ergeben bei Temperaturen von 35°C schon nach 30 Minuten intensive Färbungen mit einem großen Nuancenspektrum. Sie eignen sich deshalb besonders zum Färben von menschlichen Haaren, da sich eine Temperatur von 35°C am Kopf ohne zusätzliche Heizquelle erreichen läßt.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens ein oben unter a) näher bezeichnetes primäres aliphatisches Amin in einer Menge von 0,3 bis 65, vorzugsweise 6 bis 20 mMol, jeweils bezogen auf 100 g der gesamten Färbemittelzubereitung, und ein wasserhaltigen Träger.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens eine oben unter b) aufgeführte heterocyclische oder isocyclische aromatische Verbindung ohne primäre Aminogruppe in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens eine oben unter c) aufgeführte aromatische Carbon- oder Sulfonsäure mit primärer Aminogruppe in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens ein oben unter d) näher bezeichnetes Anilinderivat der Formel II in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens ein oben unter e) näher bezeichnetes Anilinderivat der Formel III in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens ein oben unter f) näher bezeichnetes Dianilinderivat der Formel IV in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend Isatine der Formel I in einer Menge von 0,3 bis 65, vorzugsweise 6 bis 20 mMol, mindestens eine oben unter g) näher bezeichnete nichtaromatische heterocyclische Verbindung in einer Menge von 0,3 bis 65, vorzugsweise 6 bis 20 mMol, jeweils bezogen auf 100 g der gesamten Färbemittelzubereitung, und einen wasserhaltigen Träger.

Ein weiterer Erfindungsgegenstand sind Haarfärbemittel enthaltend Isatine der Formel I in einer Menge von 0,3 bis 65, vorzugsweise 6 bis 20 mMol, einen oben unter h) aufgeführten Aminozucker in einer Menge von 0,3 bis 65, vorzugsweise 6 bis 20 mMol, jeweils bezogen auf 100 g der gesamten Färbemittelzubereitung, und einen wasserhaltigen Träger.

Wasserhaltige Träger sind z.B. Cremes, Emulsionen, Gele oder auch tensidhaltige schäumende Lösungen, wie z.B. Shampoos oder andere Zubereitungen, die für die Anwendung auf dem Haar geeignet sind.
Der wasserhaltige Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z.B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Besonders bevorzugt sind die Haarfärbemittel wenn sie zusätlich ein Salz ausgewählt aus der Gruppe der Ammonium-, Lithium-, Natrium-, Kalium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- oder Gadoliniumsalze in einer Menge von 0,3 bis 65, vorzugsweise 2 bis 15 mMol, bezogen auf 100 g der gesamten Färbemittelzubereitung, enthalten.

Der pH-Wert der Zubereitung liegt entweder im Bereich des sich für die jeweilige Verbindung a) bis h) spontan einstellenden pH-Wertes, er kann aber auch auf einen Wert zwischen 3 und 10 eingestellt werden, vorzugsweise liegt der pH-Wert bei ca. 6. Dabei ist zu beachten, daß die Farbnuance in einigen Fällen pH-abhängig ist.

Zum Haarefärben werden die erfindungsgemäßen Haarfärbemittel in Form eines wasserhaltigen kosmetischen Trägers in einer Menge von 100 g auf das Haar aufgebracht, ca. 30 Minuten dort belassen und dann ausgespült oder mit einem handelsüblichen Haarshampoo ausgewaschen.

Für die Konfektionierung der erfindungsgemäßen Haarfärbemittel gibt es mehrere Möglichkeiten.

Enthält das Haarfärbemittel lediglich die beiden Komponenten Isatinderivat und Verbindung a) bis h), so gibt es zwei Möglichkeiten der Konfektionierung, entweder beide Komponenten in einem Behälter oder beide Komponenten in getrennten Behältern. Bei Konfektionierung in getrennten Behältern können die beiden Komponenten nacheinander auf das Haar aufgetragen werden oder aber kurz vor der Anwendung zusammengemischt werden.

Enthält das Färbemittel die drei Komponenten Isatinderivat, Verbindung a) bis h) und Ammonium- oder Metallsalz, so gibt es drei Möglichkeiten der Konfektionierung, in einem, in zwei oder in drei Behältern.

In der folgenden Darstellung steht I für Isatinderivat V für Verbindung a) bis h) S für Salz:
1) Konfektionierung in einem Behälter: I + V + S
2) Konfektionierung in zwei Behältern:
   a) Behälter 1 : I + S, Behälter 2 : V
   b) Behälter 1 : V + S, Behälter 2 : I
   c) Behälter 1 : V + I, Behälter 2 : S

   Bei jeder der drei Konfektionierungsvarianten 2a), 2b) und 2c) können die Komponenten vor der Anwendung auf dem Haar zusammengemischt werden oder aber nacheinander auf das Haar aufgebracht werden.
   Bei einer getrennten Applikation auf dem Haar bestehen die beiden Möglichkeiten, zunächst den Inhalt des Behälters 1 und anschließend den Inhalt des Behälters 2 auf das Haar aufzutragen oder aber zuerst den Inhalt des Behälters 2 und anschließend den Inhalt des Behälters 1 aufzutragen.
3) Konfektionierung in drei Behältern:
   Behälter 1 : I, Behälter 2 : V, Behälter 3 : S.

Die drei Komponenten I, V und S können in einer beiliebigen Reihenfolge nacheinander auf das Haar aufgebracht werden, sie können aber auch kurz vor der Anwendung zusammengemischt werden.
Eine weitere Möglichkeit besteht darin, zunächst nur zwei der drei Komponenten I, V und S zusammenzumischen, diese Mischung auf das Haar aufzubringen, um dann erst die dritte Komponente hinzuzugeben.

### Beispiele

### Beispiel 1

### Herstellung einer Färbelösung:

Es wurde eine Aufschlämmung von 10 mMol Isatin und 1 mMol einer der in Anspruch 1 unter a) bis h) aufgeführten Komponenten in 100 ml Wasser bereitet. Die erfindungsgemäß besonders bevorzugten Zusammensetzungen enthielten zusätzlich 10 mMol Ammonium- oder Metallsalz. Die Aufschlämmung wurde auf Siedetemperatur erhitzt und nach dem Abkühlen filtriert, der pH-Wert wurde anschließend mit Salzsäure auf 6 eingestellt.
In diese Färbelösung wurden bei 35 °C 30 Minuten lang zu 90 % ergraute, nicht vorbehandelte Menschenhaare eingebracht. Die jeweiligen Färbetemperaturen, Färbedauern, Farbnuancen und Farbtiefen sind Tabelle 1 zu entnehmen.
Die Farbtiefe wurde dabei nach folgender Skala bewertet:
- -: : keine oder eine sehr blasse Ausfärbung
- (+): : schwache Intensität
- +: : mittlere Intensität
- +(+): : mittlere bis starke Intensität
- ++: : starke Intensität
- ++(+): : starke bis sehr starke Intensität
- +++: : sehr starke Intensität

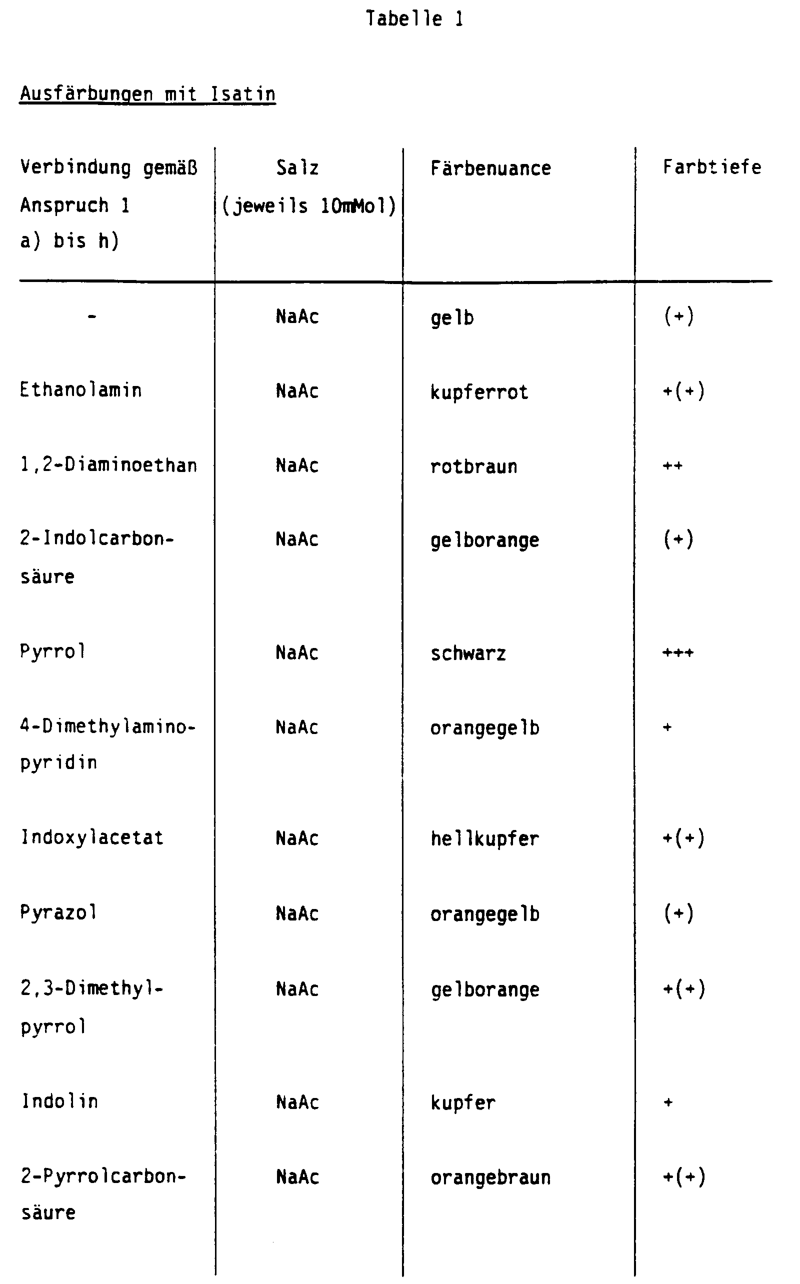

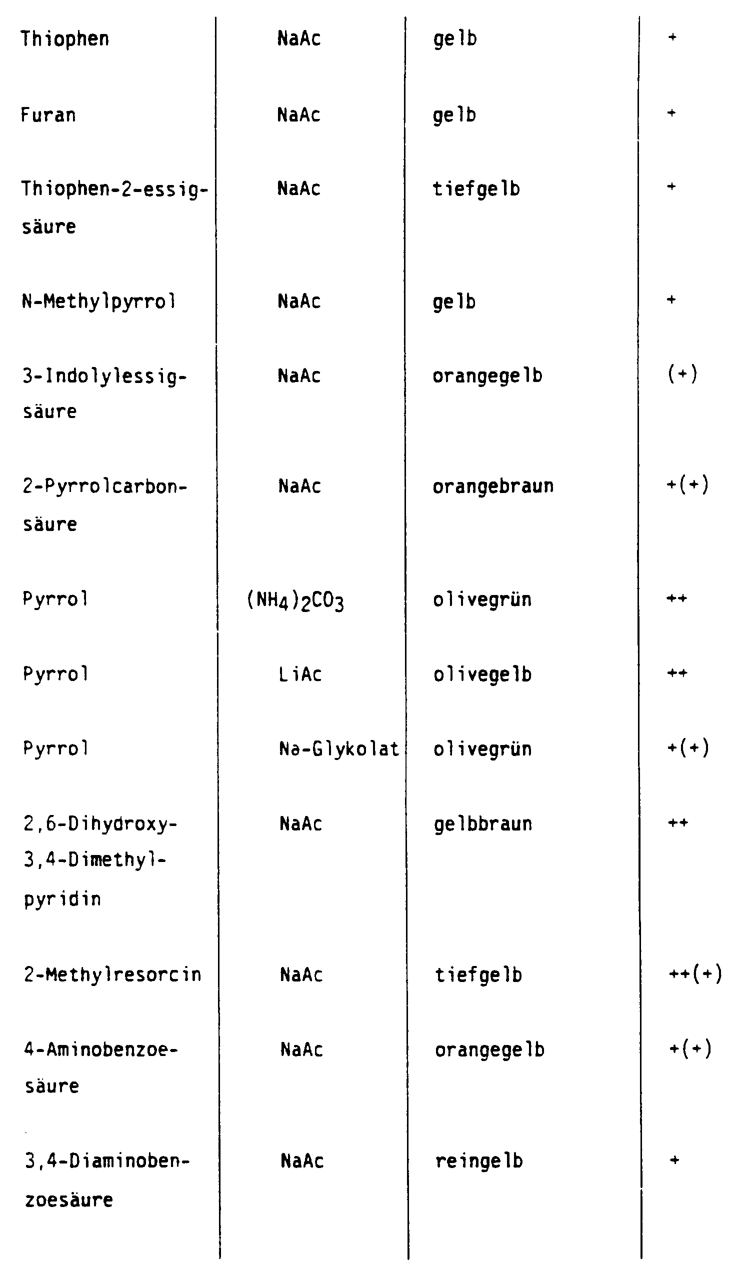

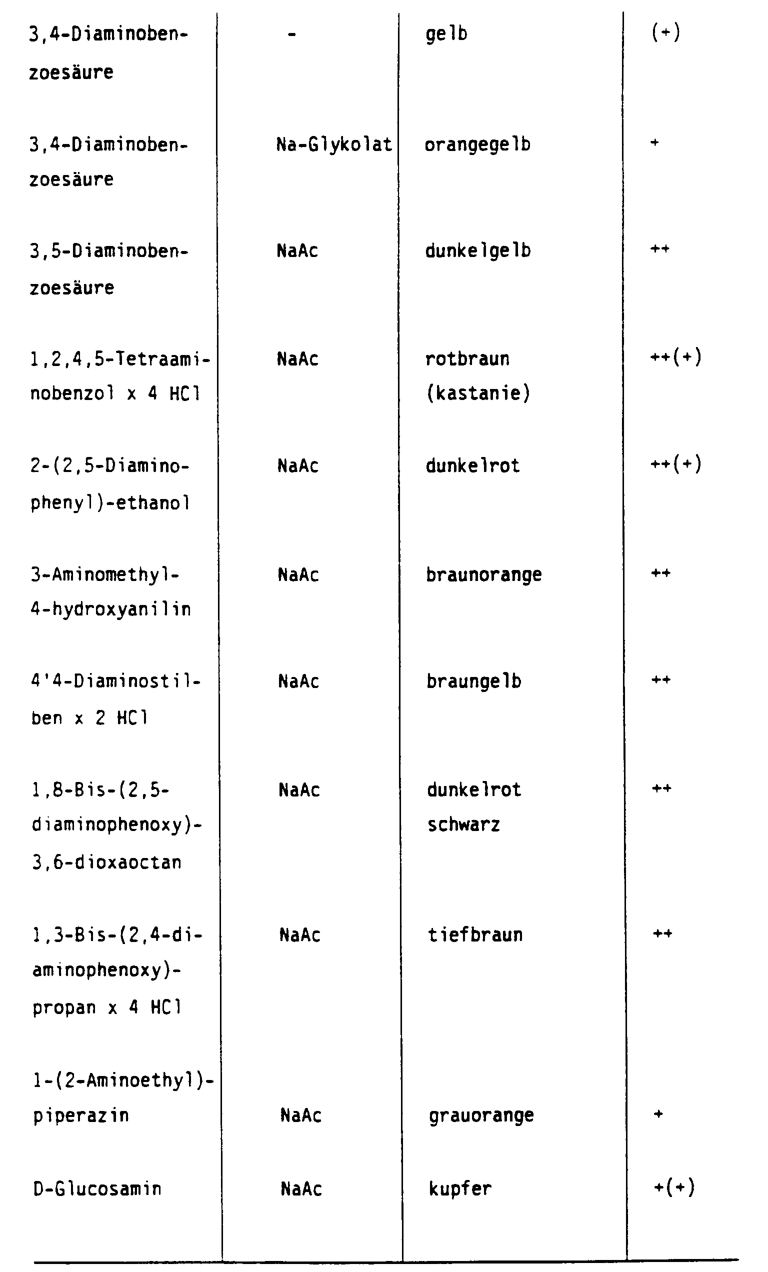

### Beispiel 2

Die Färbelösung wurde analog Beispiel 1 hergestellt, jedoch mit Isatin-5-Sulfonsäure anstelle von Isatin:

**Tabelle 2**

| Ausfärbungen mit Isatin-5-Sulfonsäure | | | |
|---|---|---|---|
| Verbindung gemäß Anspruch 1 a) bis h) | Salz (jeweils 10mMol) | Färbenuance | Farbtiefe |
| 2,4,5,6-Tetraaminopyrimidin | NaAc | kupfer | ++ |
| 1,2,4,5-Tetraaminobenzol | NaAc | olivebraun | ++ |
| 1,8 bis (2,5-Diaminophenoxy) 3,6-Dioxaoctan | NaAc | rotviolet | ++ |
| 2-(2,5-Diaminophenyl)-ethanol | NaAc | dunkelvioletrot | +++ |
| 3,4-Diaminobenzoesäure | NaAc | gelb | +(+) |
| - | NaAc | gelb | + |
| - | NaAc, ZnCl₂ | gelb | +(+) |

## Patentansprüche

1. Mittel zum Färben von keratinhaltigen Fasern enthaltend
- mindestens ein Isatinderivat der Formel I 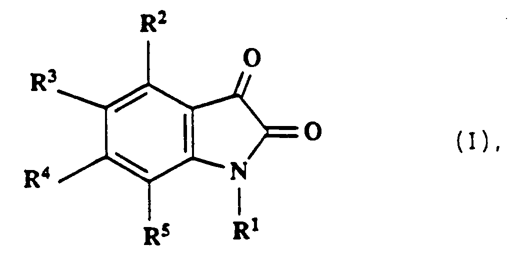 wobei R¹ Wasserstoff, eine C₁-C₄-Alkylgruppe, eine C₂-C₄-Hydroxyalkylgruppe, eine C₂-C₂₀-Acylgruppe, eine Phenylgruppe oder eine Benzoylgruppe und R², R³, R⁴ und R⁵ unabhängig voneinander Wasserstoff, Hydroxy, Halogen, Nitrogruppen, Sulfogruppen, Carboxylgruppen, C₁-C₄-Alkylgruppen, C₁-C₄-Alkoxygruppen oder NR⁶R⁷-Gruppen bedeuten, wobei R⁶ und R⁷ unabhängig voneinander Wasserstoff, C₁-C₄-Alkylgruppen oder C₂-C₄-Hydroxyalkylgruppen darstellen, und zwei benachbarte Gruppen R³, R⁴ und R⁵ auch eine Alkylendioxygruppe mit 1 bis 4 C-Atomen darstellen können,
- mindestens eine Verbindung ausgewählt aus der Gruppe
a) der primären aliphatischen Amine, die in der C-Kette mindestens eine zusätzliche Gruppe NR⁸R⁹ oder OR¹⁰ tragen, wobei R⁸, R⁹ und R¹⁰ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten,
b) der heterocyclischen oder isocyclischen aromatischen Verbindungen ohne primäre Aminogruppe,
c) der aromatischen Carbon- und Sulfonsäuren mit primärer Aminogruppe,
d) der Anilinderivate der Formel II 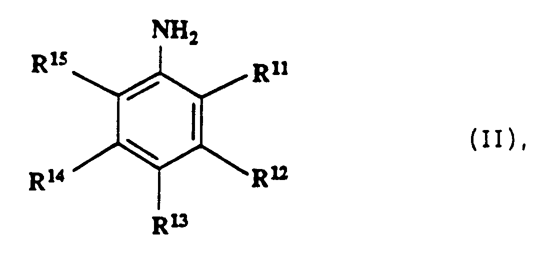 wobei R¹¹, R¹², R¹³, R¹⁴ und R¹⁵ Wasserstoff, C₁-C₄-Alkylgruppen, C₂-C₄-Hydroxyalkylgruppen, C₂-C₄-(C₁-C₄-Alkoxy)-alkylgruppen oder Gruppen NR¹⁶R¹⁷ oder OR¹⁸ darstellen, wobei R¹⁶, R¹⁷ und R¹⁸ unabhängig voneinander Wasserstoff, C₁-C₄-Alkylgruppen, C₂-C₄-Hydroxyalkylgruppen oder C₂-C₄- (C₁-C₄-Alkoxy)-alkylgruppen bedeuten, mit der Maßgabe, daß höchstens zwei der Gruppen R¹¹ bis R¹⁵ keine NR¹⁶R¹⁷- und/oder OR¹⁸-Gruppe darstellen,
e) der Anilinderivate der Formel III 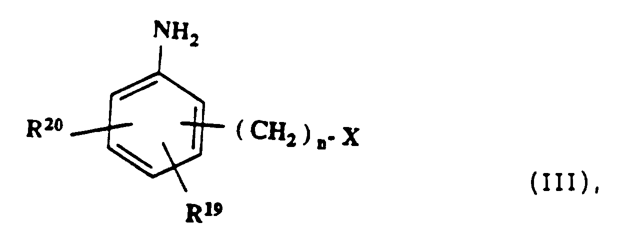 wobei n für eine ganze Zahl von 1 bis 4 und X für eine Hydroxy- oder Aminogruppe steht und R¹⁹ und R²⁰ Wasserstoff, C₁-C₄-Alkylgruppen, C₂-C₄-Hydroxyalkylgruppen, C₂-C₄-(C₁-C₄-Alkoxy)-alkylgruppen oder Gruppen NR²¹R²² oder OR²³ darstellen, wobei R²¹, R²² und R²³ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten, mit der Maßgabe, daß mindestens eine der Gruppen R¹⁹ und R²⁰ eine Gruppe NR²¹R²² oder OR²³ darstellt,
f) der Dianilinderivate der Formel IV 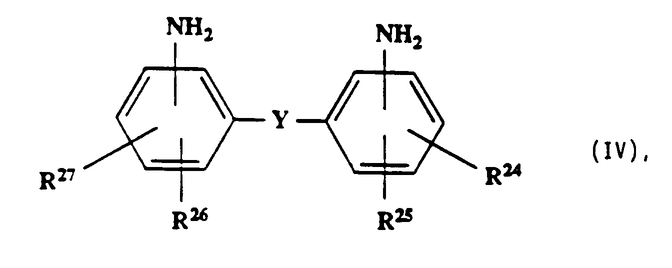 wobei Y für eine direkte Bindung oder für eine Gruppe CO, SO, O, S oder O-(CH₂-Z-CH₂-O)ₘ steht, wobei Z für eine direkte Bindung, eine Gruppe CH₂, CHOH oder CH₂OC₂H₄OCH₂ steht, und m eine ganze Zahl von 1 bis 4 bedeutet, oder Y auch eine gesättigte oder ungesättigte Alkylengruppe mit 1 bis 4 C-Atomen, die gegebenenfalls durch OH substituiert sein kann, bedeutet und R²⁴, R²⁵, R²⁶ und R²⁷ Wasserstoff, C₁-C₄-Alkylgruppen, C₂-C₄-Hydroxyalkylgruppen, C₂-C₄-(C₁-C₄-Alkoxy)-alkylgruppen oder Gruppen NR²⁸R²⁹ oder OR³⁰ darstellen, wobei R²⁸, R²⁹ und R³⁰ unabhängig voneinander Wasserstoff, C₁-C₄-Alkylgruppen, C₂-C₄-Hydroxyalkylgruppen oder C₂-C₄-(C₁-C₄-Alkoxy)-alkylgruppen bedeuten, mit der Maßgabe, daß jeweils mindestens eine der Gruppen R²⁴ und R²⁵ und eine der Gruppen R²⁶ und R²⁷ eine Gruppe NR²⁸R²⁹ oder OR³⁰ darstellt,
g) der nicht-aromatischen unsubstituierten oder mit einer der Gruppen Amino-(C₁-C₄)-alkyl, Hydroxy-(C₁-C₄)-alkyl oder Carboxyl substituierten Heterocyclen,
h) der Aminozucker
- und einen wasserhaltigen Träger.

2. Mittel zum Färben von keratinhaltigen Fasern nach Anspruch 1, dadurch gekennzeichnet, daß im Isatinderivat der Formel I R¹ Wasserstoff ist und R², R³, R⁴ und R⁵ unabhängig voneinander Wasserstoff, Hydroxy, Halogen, Methyl-, Sulfogruppen oder NR⁶R⁷-Gruppen darstellen, worin R⁶ und R⁷ Wasserstoff bedeuten.

3. Mittel zum Färben von keratinhaltigen Fasern nach Anspruch 1 und 2, dadurch gekennzeichnet, daß die Verbindung der Formel I Isatin ist.

4. Mittel zum Färben von keratinhaltigen Fasern nach Anspruch 1 bis 3, dadurch gekennzeichnet, daß die im Anspruch 1 unter a) näher bezeichneten primären aliphatischen Amine ausgewählt sind aus 2-Aminoethanol, 2-Methoxyamin, 2-Ethoxyethylamin, 2-(2-Aminoethoxy)-ethanol, 2-Aminopropanol, 3-Aminopropanol, 2,3-Dihydroxypropylamin, 4-Hydroxypropylamin, 2-Aminopropan-1,3-diol, 2-Amino-2-methylpropanol, 2-Amino-2-methylpropan-1,3-diol, 2-Amino-2-hydroxymethylpropan-1,3-diol, Tetrahydroxypentylaminen, Pentahydroxyhexylaminen, 1,2-Diaminoethan, 1,2-Diaminopropan, 1,3-Diaminopropan, 1,3-Diamino-2-propanol, 2-(2-Aminoethylamino)-ethylamin, 2-(2-Aminoethylamino)-ethanol, 3-(2-Aminoethylamino)-propylamin, 3-(2-Aminoethylamino)-propanol.

5. Mittel zum Färben von keratinhaltigen Fasern nach Anspruch 1 bis 3, dadurch gekennzeichnet, daß die heterocyclischen oder isocyclischen aromatischen Verbindungen ohne primäre Aminogruppe ausgewählt sind aus Indolin, Indol, Pyrrol, 1-Methylpyrrol, 2-Methylpyrrol, 3-Methylpyrrol, 2,5-Dimethylpyrrol, Pyrazol, 3-Methylpyrazol, Imidazol, Indoxylacetat, Tetrahydrochinolin, Tetrahydroisochinolin, 2-Indolcarbonsäure, 3-Indolylessigsäure, 4-Dimethylaminopyridin, 2,6-Dihydroxy-3,4-dimethylpyridin, Pyrrol-2-carbonsäure, 2-Methylresorcin.

6. Mittel zum Färben von keratinhaltigen Fasern nach Anspruch 1 bis 3, dadurch gekennzeichnet, daß aromatischen Carbon- und Sulfonsäuren mit primärer Aminogruppe ausgewählt sind aus 2-, 3- und 4-Aminobenzoesäure, 2-, 3- und 4-Phenylessigsäure, 2,3-, 2,4-, 2,5-, 3,4- und 3,5-Diaminobenzoesäure, 4- oder 5-Aminosalicylsäure, 3-Amino-4-hydroxybenzoesäure, 4-Amino-3-hydroxybenzoesäure, 2-, 3- und 4-Aminobenzolsulfonsäure, 4-Amino-3-hydroxynaphthalin-1-sulfonsäure, 3-Aminophthalsäure, 5-Aminoisophthalsäure.

7. Mittel zum Färben von keratinhaltigen Fasern nach Anspruch 1 bis 3, dadurch gekennzeichnet, daß die Anilinderivate der Formel II ausgewählt sind aus 1,2,4,5-Tetraaminobenzol, 2,4,5-Triaminophenol, Pentaaminobenzol, Hexaaminobenzol, 2,4,6-Triaminoresorcin, 4,5-Diaminobrenzkatechin, 4,6-Diaminopyrogallol, 2,5-Dihydroxy-4-morpholinoanilin, 3,5-Diamino-4-hydroxybrenzkatechin.

8. Mittel zum Färben von keratinhaltigen Fasern nach Anspruch 1 bis 3, dadurch gekennzeichnet, daß die Anilinderivate der Formel III ausgewählt sind aus 2-(2,5-Diaminophenyl)-ethanol, 3-Aminomethyl-4-aminophenol, 3-(2,5-Diaminophenyl)-propanol, 2-Aminomethyl-4-aminophenol, 2-Aminomethyl-5-aminophenol, 2-(2,4-Diaminophenyl)ethanol.

9. Mittel zum Färben von keratinhaltigen Fasern nach Anspruch 1 bis 3, dadurch gekennzeichnet, daß die Dianiline der Formel IV ausgewählt sind aus 4,4'-Diaminostilben, 4,4'Diaminostilben-2,2'-disulfonsäure, Natriumsalz, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminobenzophenon, 4,4'-Diaminobenzodiphenylether, 3,3',4,4'-Tetraaminodiphenyl, 3,3', 4,4'-Tetraaminobenzophenon, 1,3-Bis-(2,4-diaminophenoxy)-propan, 1,8-Bis-(2,5-diaminophenoxy)-3,6-dioxaoctan.

10. Mittel zum Färben von keratinhaltigen Fasern nach Anspruch 1 bis 3, dadurch gekennzeichnet, daß die nicht-aromatischen unsubstituierten oder mit einer der Gruppen Amino-(C₁-_{C4})-alkyl, Hydroxy-(C₁-C₄)-alkyl oder Arboxyl substituierten Heterocyclen ausgewählt sind aus Piperidin, Piperazin, 1-(2-Aminooctyl)-piperazin, 1-(2-Hydroxyethyl)-piperazin, 3-Pyrrolin, Pyrrolidin, Thiazolidin, Thiazolidin-4-carbonsäure, Piperidin-2-carbonsäure, Piperidin-3-carbonsäure, Piperidin-4-carbonsäure.

11. Mittel zum Färben von keratinhaltigen Fasern nach Anspruch 1 bis 3, dadurch gekennzeichnet, daß der Aminozucker ausgewählt ist aus D-Glucosamin und D-Galactosamin.

12. Mittel zum Färben von keratinhaltigen Fasern nach Anspruch 1 bis 11, dadurch gekennzeichnet, daß mindestens ein Salz ausgewählt aus der Gruppe der Ammonium-, Lithium-, Natrium-, Kalium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze enthalten ist.

13. Mittel zum Färben von keratinhaltigen Fasern nach Anspruch 12, dadurch gekennzeichnet, daß das Salz ausgewählt ist aus der Gruppe Ammoniumcarbonat, Ammoniumacetat, Natriumacetat, Lithiumacetat, Kaliumacetat, Natriumglykolat, Natriumlactat, Calciumgluconat.

14. Haarfärbemittel enthaltend mindestens ein Isatin der Formel I nach Anspruch 1 in einer Menge von 0,3 bis 65, vorzugsweise 6 bis 20 mmol, mindestens ein primäres aliphatisches Amin, das in der C-Kette mindestens eine zusätzliche Gruppe NR⁸R⁹ oder OR¹⁰ trägt, in einer Menge von 0,3 bis 65, vorzugsweise 6 bis 20 mmol, jeweils bezogen auf 100 g der gesamten Färbemittelzubereitung, und einen wasserhaltigen Träger.

15. Haarfärbemittel enthaltend mindestens ein Isatin der Formel nach Anspruch 1 I in einer Menge von 0,3 bis 65, vorzugsweise 6 bis 20 mmol, mindestens eine heterocyclische oder isocyclische aromatische Verbindung ohne primäre Amingruppe in einer Menge von 0,3 bis 65, vorzugsweise 6 bis 20 mmol, jeweils bezogen auf 100 g der gesamten Färbemittelzubereitung, und einen wasserhaltigen Träger.

16. Haarfärbemittel enthaltend mindestens ein Isatin der Formel I nach Anspruch 1 in einer Menge von 0,3 bis 65, vorzugsweise 6 bis 20 mmol, mindestens eine aromatische Carbon- oder Sulfonsäure mit primärer Aminogruppe in einer Menge von 0,3 bis 65, vorzugsweise 6 bis 20 mmol, jeweils bezogen auf 100 g der gesamten Färbemittelzubereitung, und einen wasserhaltigen Träger.

17. Haarfärbemittel enthaltend mindestens ein Isatin der Formel I nach Anspruch 1 in einer Menge von 0,3 bis 65, vorzugsweise 6 bis 20 mmol, mindestens ein Anilinderivat der Formel II in einer Menge von 0,3 bis 65, vorzugsweise 6 bis 20 mmol, jeweils bezogen auf 100 g der gesamten Färbemittelzubereitung, und einen wasserhaltigen Träger.

18. Haarfärbemittel enthaltend mindestens ein Isatin der Formel I nach Anspruch 1 in einer Menge von 0,3 bis 65, vorzugsweise 6 bis 20 mmol, mindestens ein Anilinderivat der Formel III in einer Menge von 0,3 bis 65, vorzugsweise 6 bis 20 mmol, jeweils bezogen auf 100 g der gesamten Färbemittelzubereitung, und einen wasserhaltigen Träger.

19. Haarfärbemittel enthaltend mindestens ein Isatin der Formel I nach Anspruch 1 in einer Menge von 0,3 bis 65, vorzugsweise 6 bis 20 mmol, mindestens ein Dianilinderivat der Formel IV in einer Menge von 0,3 bis 65, vorzugsweise 6 bis 20 mmol, jeweils bezogen auf 100 g der gesamten Färbemittelzubereitung, und einen wasserhaltigen Träger.

20. Haarfarbemittel enthaltend mindestens ein Isatin der Formel I nach Anspruch 1 in einer Menge von 0,3 bis 65, vorzugsweise 6 bis 20 mmol, mindestens eine nicht-aromatische unsubstituierte oder mit einer der Gruppen Amino-(C₁-C₄)-alkyl, Hydroxy-(C₁-C₄)-alkyl oder Carboxyl substituierten heterocyclischen Verbindung in einer Menge von 0,3 bis 65, vorzugsweise 6 bis 20 mmol, jeweils bezogen auf 100 g der gesamten Färbemittelzubereitung, und einen wasserhaltigen Träger.

21. Haarfärbemittel enthaltend mindestens ein Isatin der Formel I nach Anspruch 1 in einer Menge von 0,3 bis 65, vorzugsweise 6 bis 20 mmol, mindestens einen Aminozucker in einer Menge von 0,3 bis 65, vorzugsweise 6 bis 20 mmol, jeweils bezogen auf 100 g der gesamten Färbemittelzubereitung, und einen wasserhaltigen Träger.

22. Haarfärbemittel nach Anspruch 14 bis 21 enthaltend zusätzlich ein Salz ausgewählt aus der Gruppe der Ammonium-, Lithium-, Natrium-, Kalium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze in einer Menge von 0,3 bis 65, vorzugsweise 2 bis 15 mmol, bezogen auf 100 g der gesamten Färbemittelzubereitung.

## Claims

1. Formulations for coloring keratin-containing fibers containing
- at least one isatin derivative corresponding to formula I: 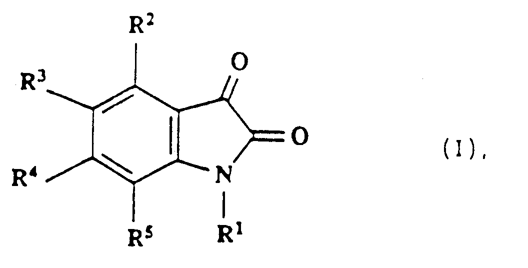 in which R¹ is hydrogen, a C₁₋₄ alkyl group, a C₂₋₄ hydroxyalkyl group, a C₂₋₂₀ acyl group, a phenyl group or a benzoyl group and R², R³, R⁴ and R⁵ independently of one another represent hydrogen, hydroxy, halogen, nitro groups, sulfo groups, carboxyl groups, C₁₋₄ alkyl groups, C₁₋₄ alkoxy groups or NR⁶R⁷ groups where R⁶ and R⁷ independently of one another represent hydrogen, C₁₋₄ alkyl groups or C₂₋₄ hydroxyalkyl groups; two adjacent groups R³, R⁴ and R⁵ may also represent an alkylenedioxy group containing 1 to 4 carbon atoms,
- at least one compound selected from the group of
a) primary aliphatic amines which contain at least one additional group NR⁸R⁹ or OR¹⁰ in the C chain, R⁸, R⁹ and R¹⁰ independently of one another representing hydrogen, C₁₋₄ alkyl groups, C₂₋₄ hydroxyalkyl groups or C₂₋₄-(C₁₋₄-alkoxy)-alkyl groups,
b) heterocyclic or isocyclic aromatic compounds without a primary amino group,
c) aromatic carboxylic and sulfonic acids with a primary amino group.
d) aniline derivatives corresponding to formula II: 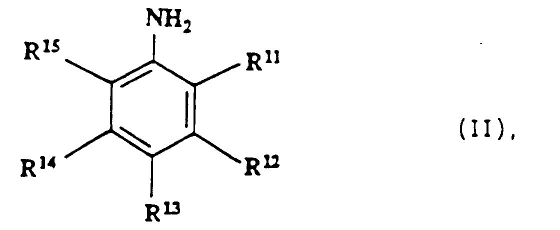 in which R¹¹, R¹², R¹³, R¹⁴ and R¹⁵ represent hydrogen, C₁₋₄ alkyl groups, C₂₋₄ hydroxyalkyl groups, C₂₋₄-(C₁₋₄ alkoxy)-alkyl groups or groups NR¹⁶R¹⁷ or OR¹⁸, where R¹⁶, R¹⁷ and R¹⁸ independently of one another represent hydrogen, C₁₋₄ alkyl groups, C₂₋₄ hydroxyalkyl groups or C₂₋₄-(C₁₋₄ alkoxy)-alkyl groups, with the proviso that at most two of the groups R¹¹ to R¹⁵ are not an NR¹⁶R¹⁷ and/or OR¹⁸ group,
e) aniline derivatives corresponding to formula III: 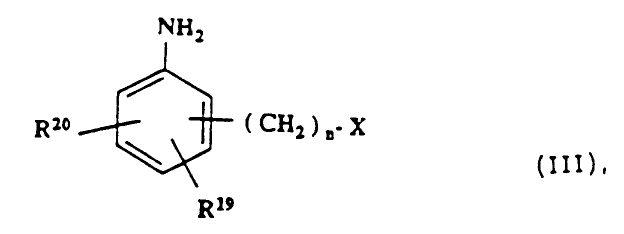 in which n is an integer of 1 to 4 and X is a hydroxy or amino group and R¹⁹ and R²⁰ represent hydrogen, C₁₋₄ alkyl groups, C₂₋₄ hydroxyalkyl groups, C₂₋₄-(C₁₋₄ alkoxy)-alkyl groups or groups NR²¹R²² or OR²³, where R²¹, R²² and R²³ independently of one another represent hydrogen, C₁₋₄ alkyl groups, C₂₋₄ hydroxyalkyl groups or C₂₋₄-(C₁₋₄ alkoxy)-alkyl groups, with the proviso that at least one of the groups R¹⁹ and R²⁰ is a group NR²¹R²² or OR²³,
f) dianiline derivatives corresponding to formula IV: 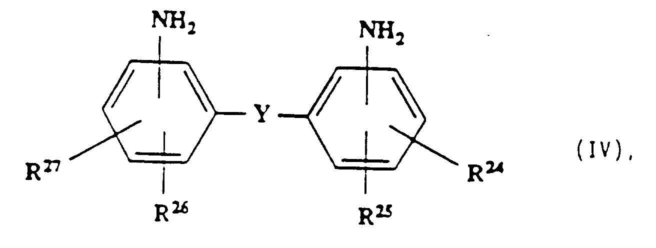 in which Y is a direct bond or a group CO, SO, O, S or O-(CH₂-Z-CH₂-O)ₘ, where Z is a direct bond, a group CH₂, CHOH or CH₂OC₂H₄OCH₂ and m is an integer of 1 to 4, or Y may even be a saturated or unsaturated alkylene group containing 1 to 4 carbon atoms which may optionally be substituted by OH and R²⁴, R²⁵, R²⁶ and R²⁷ represent hydrogen, C₁₋₄ alkyl groups, C₂₋₄ hydroxyalkyl groups, C₂₋₄-(C₁₋₄ alkoxy)-alkyl groups or groups NR²⁸R²⁹ or OR³⁰, where R²⁸, R²⁹ and R³⁰ independently of one another represent hydrogen, C₁₋₄ alkyl groups, C₂₋₄ hydroxyalkyl groups or C₂₋₄-(C₁₋₄ alkoxy)-alkyl groups, with the proviso that at least one of the groups R²⁴ and R²⁵ and one of the groups R²⁶ and R²⁷ is a group NR²⁸R²⁹ or OR³⁰,
g) non-aromatic unsubstituted or amino-(C₁₋₄)-alkyl-, hydroxy-(C₁₋₄)-alkyl- or carboxyl-substituted heterocycles,
h) amino sugars,
- and a water-containing carrier.

2. Formulations for coloring keratin-containing fibers as claimed in claim 1, characterized in that, in the isatin derivative corresponding to formula I, R¹ is hydrogen and R², R³, R⁴ and R⁵ independently of one another represent hydrogen, hydroxy, halogen, methyl, sulfo groups or NR⁶R⁷ groups, where R⁶ and R⁷ are hydrogen.

3. Formulations for coloring keratin-containing fibers as claimed in claims 1 and 2, characterized in that the compound corresponding to formula I is isatin.

4. Formulations for coloring keratin-containing fibers as claimed in claims 1 to 3, characterized in that the primary aliphatic amines defined above under a) in claim 1 are selected from 2-aminoethanol, 2-methoxyamine, 2-ethoxyethylamine, 2-(2-aminoethoxy)-ethanol, 2-aminopropanol, 3-aminopropanol, 2,3-dihydroxypropylamine, 4-hydroxypropylamine, 2-aminopropane-1,3-diol, 2-amino-2-methyl propanol, 2-amino-2-methyl propane-1,3-diol, 2-amino-2-hydroxymethyl propane-1,3-diol, tetrahydroxypentylamines, pentahydroxyhexylamines, 1,2-diaminoethane, 1,2-diaminopropane, 1,3-diaminopropane, 1,3-diamino-2-propanol, 2-(2-aminoethylamino)-ethylamine, 2-(2-aminoethylamino)-ethanol, 3-(2-aminoethylamino)-propylamine, 3-(2-aminoethylamino)-propanol.

5. Formulations for coloring keratin-containing fibers as claimed in claims 1 to 3, characterized in that the heterocyclic or isocyclic aromatic compounds with no primary amino group are selected from indoline, indole, pyrrole, 1-methyl pyrrole, 2-methyl pyrrole, 3-methyl pyrrole, 2,5-dimethyl pyrrole, pyrazole, 3-methyl pyrazole, imidazole, indoxyl acetate, tetrahydroquinoline, tetrahydroisoquinoline, 2-indole carboxylic acid, 3-indolyl acetic acid, 4-dimethyl aminopyridine, 2,6-dihydroxy-3,4-dimethyl pyridine, pyrrole-2-carboxylic acid, 2-methyl resorcinol.

6. Formulations for coloring keratin-containing fibers as claimed in claims 1 to 3, characterized in that the aromatic carboxylic and sulfonic acids containing a primary amino group are selected from 2-, 3- and 4-aminobenzoic acid, 2-, 3- and 4-phenyl acetic acid, 2,3-, 2,4-, 2,5-, 3,4- and 3,5-diaminobenzoic acid, 4- or 5-aminosalicylic acid, 3-amino-4-hydroxybenzoic acid, 4-amino-3-hydroxybenzoic acid, 2-, 3- and 4-aminobenzene sulfonic acid, 4-amino-3-hydroxynaphthalene-1-sulfonic acid, 3-aminophthalic acid, 5-aminoisophthalic acid.

7. Formulations for coloring keratin-containing fibers as claimed in claims 1 to 3, characterized in that the aniline derivatives of formula II are selected from 1,2,4,5-tetraaminobenzene, 2,4,5-triaminophenol, pentaaminobenzene, hexaaminobenzene, 2,4,6-triaminoresorcinol, 4,5-diaminopyrocatechol, 4,6-diaminopyrogallol, 2,5-dihydroxy-4-morpholinoaniline, 3,5-diamino-4-hydroxypyrocatechol.

8. Formulations for coloring keratin-containing fibers as claimed in claims 1 to 3, characterized in that the aniline derivatives of formula III are selected from 2-(2,5-diaminophenyl)-ethanol, 3-aminomethyl-4-aminophenol, 3-(2,5-diaminophenyl)-propanol, 2-aminomethyl-4-aminophenol, 2-aminomethyl-5-aminophenol, 2-(2,4-diaminophenyl)-ethanol.

9. Formulations for coloring keratin-containing fibers as claimed in claims 1 to 3, characterized in that the dianiline derivatives of formula IV are selected from 4,4'-diaminostilbene, 4,4'-diaminostilbene-2,2'-disulfonic acid, sodium salt, 4,4'-diaminodiphenyl methane, 4,4'-diaminodiphenyl sulfide, 4,4'-diaminodiphenyl sulfoxide, 4,4'-diaminodiphenylamine, 4,4'-diaminobenzophenone, 4,4'-diaminobenzodiphenylether, 3,3',4,4'-tetraaminodiphenyl, 3,3',4,4'-tetraaminobenzophenone, 1,3-bis-(2,4-diaminophenoxy)-propane, 1,8-bis-(2,5-diaminophenoxy)-3,6-dioxaoctane.

10. Formulations for coloring keratin-containing fibers as claimed in claims 1 to 3, characterized in that the non-aromatic, unsubstituted or amino-(C₁₋₄)-alkyl, hydroxy-(C₁₋₄)-alkyl or carboxyl-substituted heterocycles are selected from piperidine, piperazine, 1-(2-aminooctyl)-piperazine, 1-(2-hydroxyethyl)-piperazine, 3-pyrroline, pyrrolidine, thiazolidine, thiazolidine-4-carboxylic acid, piperidine-2-carboxylic acid, piperidine-3-carboxylic acid, piperidine-4-carboxylic acid.

11. Formulations for coloring keratin-containing fibers as claimed in claims 1 to 3, characterized in that the amino sugar is selected from D-glucosamine and D-galactosamine.

12. Formulations for coloring keratin-containing fibers as claimed in claims 1 to 11, characterized in that at least one salt is selected from the group consisting of ammonium, lithium, sodium, potassium, magnesium, calcium, strontium, barium, aluminium, titanium, manganese, iron, cobalt, nickel, copper, silver, zinc, lanthanum, cerium, praseodymium, neodymium and gadolinium salts.

13. Formulations for coloring keratin-containing fibers as claimed in claim 12, characterized in that the salt is selected from the group consisting of ammonium carbonate, ammonium acetate, sodium acetate, lithium acetate, potassium acetate, sodium glycolate, sodium lactate, calcium gluconate.

14. Hair coloring formulations containing at least one isatin corresponding to formula I in claim 1 in a quantity of 0.3 to 65 mmoles and preferably 6 to 20 mmoles, at least one primary aliphatic amine containing at least one additional group NR⁸R⁹ or OR¹⁰ in the C chain in a quantity of 0.3 to 65 mmoles and preferably 6 to 20 mmoles, based on 100 g of the coloring formulation as a whole, and a water-containing carrier.

15. Hair coloring formulations containing at least one isatin corresponding to formula I in claim 1 in a quantity of 0.3 to 65 mmoles and preferably 6 to 20 mmoles, at least one heterocyclic or isocyclic aromatic compound with no primary amino group in a quantity of 0.3 to 65 mmoles and preferably 6 to 20 mmoles, based on 100 g of the coloring formulation as a whole, and a water-containing carrier.

16. Hair coloring formulations containing at least one isatin corresponding to formula I in claim 1 in a quantity of 0.3 to 65 mmoles and preferably 6 to 20 mmoles, at least one aromatic carboxylic or sulfonic acid with a primary amino group in a quantity of 0.3 to 65 mmoles and preferably 6 to 20 mmoles, based on 100 g of the coloring formulation as a whole, and a water-containing carrier.

17. Hair coloring formulations containing at least one isatin corresponding to formula I in claim 1 in a quantity of 0.3 to 65 mmoles and preferably 6 to 20 mmoles, at least one aniline derivative corresponding to formula II in a quantity of 0.3 to 65 mmoles and preferably 6 to 20 mmoles, based on 100 g of the coloring formulation as a whole, and a water-containing carrier.

18. Hair coloring formulations containing at least one isatin corresponding to formula I in claim 1 in a quantity of 0.3 to 65 mmoles and preferably 6 to 20 mmoles, at least one aniline derivative corresponding to formula III in a quantity of 0.3 to 65 mmoles and preferably 6 to 20 mmoles, based on 100 g of the coloring formulation as a whole, and a water-containing carrier.

19. Hair coloring formulations containing at least one isatin corresponding to formula I in claim 1 in a quantity of 0.3 to 65 mmoles and preferably 6 to 20 mmoles, at least one dianiline derivative corresponding to formula IV in a quantity of 0.3 to 65 mmoles and preferably 6 to 20 mmoles, based on 100 g of the coloring formulation as a whole, and a water-containing carrier.

20. Hair coloring formulations containing at least one isatin corresponding to formula I in claim 1 in a quantity of 0.3 to 65 mmoles and preferably 6 to 20 mmoles, at least one non-aromatic, unsubstituted or amino-(C₁₋₄)-alkyl, hydroxy-(C₁₋₄)-alkyl or carboxyl-substituted compound in a quantity of 0.3 to 65 mmoles and preferably 6 to 20 mmoles, based on 100 g of the coloring formulation as a whole, and a water-containing carrier.

21. Hair coloring formulations containing at least one isatin corresponding to formula I in claim 1 in a quantity of 0.3 to 65 mmoles and preferably 6 to 20 mmoles, at least one amino sugar in a quantity of 0.3 to 65 mmoles and preferably 6 to 20 mmoles, based on 100 g of the coloring formulation as a whole, and a water-containing carrier.

22. Hair coloring formulations as claimed in claims 14 to 21 additionally containing a salt selected from the group consisting of ammonium, lithium, sodium, potassium, magnesium, calcium, strontium, barium, aluminium, titanium, manganese, iron, cobalt, nickel, copper, silver, zinc, lanthanum, cerium, praseodymium, neodymium and gadolinium salts in a quantity of 0.3 to 65 and preferably 2 to 15 mmoles based on 100 g of the coloring formulation as a whole.

## Revendications

1. Agents permettant de colorer des fibres contenant de la kératine renfermant
- au moins un dérivé d'isatine de la formule I 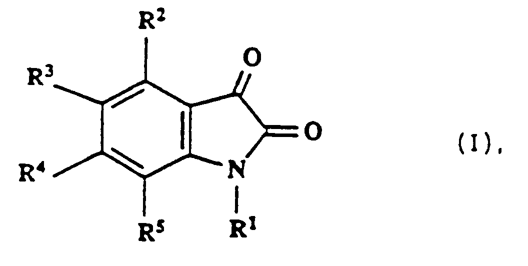 dans laquelle R¹ représente l'hydrogène, un groupe alkyle en C₁-C₄, un groupe hydroxyalkyle en C₂-C₄, un groupe acyle en C₂-C₂₀, un groupe phényle ou un groupe benzoyle, et R², R³, R⁴ et R⁵ représentent, chacun, indépendamment les uns des autres, l'hydrogène, un groupe hydroxyle, un halogène, un groupe nitro, sulfo, carboxyle, alkyle en C₁-C₄, alcoxy en C₁-C₄ ou un groupe NR⁶R⁷, dans lequel R⁶ et R⁷ peuvent représenter, chacun, indépendamment l'un de l'autre, l'hydrogène, un groupe alkyle en C₁-C₄ ou hydroxyalkyle en C₂-C₄, et deux groupes R³, R⁴ et R⁵ voisins peuvent également représenter un groupe alkylènedioxy comportant 1 à 4 atomes de C.
- au moins un composé sélectionné parmi le groupe
a) des amines aliphatiques primaires, qui portent dans la chaîne C au moins un groupe NR⁸R⁹ ou OR¹⁰ supplémentaire, dans lequel R⁸, R⁹ et R¹⁰ représentent, chacun, indépendamment les uns des autres, l'hydrogène, un groupe alkyle en C₁-C₄, un groupe hydroxyalkyle en C₂-C₄ ou un groupe (alcoxy en C₁-C₄) alkyle en C₂-C₄,
b) des composés aromatiques hétérocycliques ou isocycliques sans groupe amino primaire,
c) des acides carboxyliques et sulfoniques aromatiques comportant un groupe amino primaire,
d) des dérivés d'aniline de la formule II 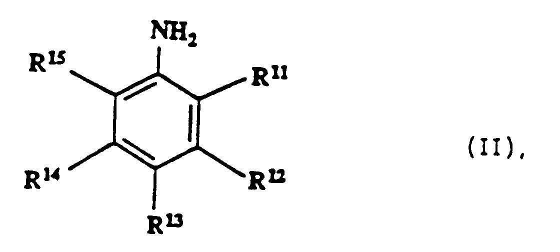 dans laquelle R¹¹, R¹², R¹³, R¹⁴ et R¹⁵ représentent chacun l'hydrogène, un groupe alkyle en C₁-C₄, un groupe hydroxyalkyle en C₂-C₄, un groupe (alcoxy en C₁-C₄) alkyle en C₂-C₄ ou un groupe NR¹⁶R¹⁷ ou OR¹⁸, dans lequel R¹⁶, R¹⁷ et R¹⁸ représentent, chacun, indépendamment les uns des autres, l'hydrogène, un groupe alkyle en C₁-C₄, un groupe hydroxyalkyle en C₂-C₄ ou un groupe (alcoxy en C₁-C₄) alkyle en C₂-C₄, à condition qu'au maximum deux des groupes R¹¹ à R¹⁵ ne représentent pas de groupe NR¹⁶R¹⁷ et/ou de groupe OR¹⁸.
e) des dérivés d'aniline de la formule III 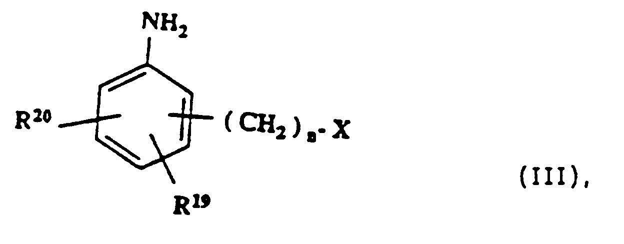 dans laquelle n représente un nombre entier de 1 à 4, X correspond à un groupe hydroxyle ou amino et R¹⁹ et R²⁰ représentent chacun l'hydrogène, un groupe alkyle en C₁-C₄, un groupe hydroxyalkyle en C₂-C₄, un groupe (alcoxy en C₁-C₄) alkyle en C₂-C₄ ou un groupe NR²¹R²² ou OR²³, dans lequel R²¹, R²² et R²³ représentent, chacun, indépendamment les uns des autre, l'hydrogène, un groupe alkyle en C₁-C₄, un groupe hydroxyalkyle en C₂-C₄ ou un groupe (alcoxy en C₁-C₄) alkyle en C₂-C₄, à condition qu'au moins un des groupes R¹⁹ à R²⁰ représente un groupe NR²¹R²² ou OR²³.
f) des dérivés de dianiline de la formule IV 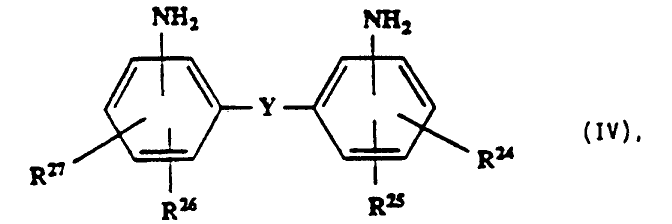 dans laquelle Y représente une liaison directe ou un groupe CO, SO, O, S ou O-(CH₂-Z-CH₂-O)ₘ, dans lequel Z correspond à une liaison directe, à un groupe CH₂, CHOH ou CH₂OC₂H₄OCH₂, et m est un nombre entier de 1 à 4, ou Y peut également représenter un groupe alkylène saturé ou insaturé comportant 1 à 4 atomes de C, qui peut être éventuellement substitué par OH, et R²⁴, R²⁵, R²⁶ et R²⁷ représentent l'hydrogène, un groupe alkyle en C₁-C₄, un groupe hydroxyalkyle en C₂-C₄, un groupe (alcoxy en C₁-C₄) alkyle en C₂-C₄ ou un groupe NR²⁸R²⁹ ou OR³⁰, dans lequel R²⁸, R²⁹ et R³⁰ représentent, chacun, indépendamment les uns des autres, l'hydrogène, un groupe alkyle en C₁-C₄, un groupe hydroxyalkyle en C₂-C₄ ou un groupe (alcoxy en C₁-C₄) alkyle en C₂-C₄, à condition qu'au moins un des groupes R²⁴ et R²⁵ et un des groupes R²⁶ et R²⁷ représentent un groupe NR²⁸R²⁹ ou OR³⁰.
g) des hétérocycles non aromatiques non substitués ou substitués par un des groupes amino-(alkyle en C₁-C₄), hydroxy-(alkyle en C₁-C₄) ou carboxyle,
h) des hexosamines
- et un vecteur aqueux.

2. Agents permettant de colorer des fibres contenant de la kératine selon la revendication 1, caractérisés en ce que dans le dérivé d'isatine de la formule I, R¹ représente l'hydrogène et R², R³, R⁴ et R⁵ correspondent indépendamment les uns des autres à l'hydrogène, à un groupe hydroxyle , à un halogène, à des groupes méthyle, sulfo ou à un groupe NR⁶R⁷, dans lequel R⁶ et R⁷ représentent l'hydrogène.

3. Agents permettant de colorer des fibres contenant de la kératine selon la revendication 1 à 2 caractérisés en ce que le composé de la formule I est l'isatine.

4. Agents permettant de colorer des fibres contenant de la kératine selon la revendication 1 à 3, caractérisés en ce que les amines aliphatiques primaires définies plus précisément en a) dans la revendication 1 sont sélectionnées parmi les composés suivants: 2-aminoéthanol, 2-méthoxyamine, 2-éthoxyéthylamine, 2-(2-aminoéthoxy)-éthanol, 2-aminopropanol, 3-aminopropanol, 2,3-dihydroxypropylamine, 4-hydroxypropylamine, 2-aminopropane-1,3-diol, 2-amino-2-méthylpropanol, 2-amino-2-méthylpropane-1,3-diol, 2-amino-2-hydroxyméthylpropane-1,3-diol, tétrahydroxypentylamines, pentahydroxyhexylamines, 1,2-diaminoéthane, 1,2-diaminopropane, 1,3-diaminopropane, 1,3-diamino-2-propanol, 2-(2-aminoéthylamino)-éthylamine, 2-(2-aminoéthylamino)-éthanol, 3-(2-aminoéthylamino)-propylamine, 3-(2-aminoéthylamino)-propanol.

5. Agents permettant de colorer des fibres contenant de la kératine selon les revendications 1 à 3, caractérisés en ce que les composés aromatiques hétérocycliques ou isocycliques sans groupe amino primaire sont sélectionnés parmi les composés suivants: indoline, indole, pyrrol, 1-méthylpyrrol, 2-méthylpyrrol, 3-méthylpyrrol, 2,5-diméthylpyrrol, pyrazol, 3-méthylpyrazol, imidazol, acétate d'indoxyle, tétrahydroquinoléine, tétrahydroisoquinoléine, acide 2-indolcarboxylique, acide 3-indolylacétique, 4-diméthylaminopyridine, 2,6-dihydroxy-3,4-diméthylpyridine, acide pyrrol-2-carboxylique, 2-méthylrésorcine.

6. Agents permettant de colorer des fibres contenant de la kératine selon les revendications 1 à 3, caractérisés en ce que les acides carboxyliques et sulfoniques aromatiques comportant un groupe amino primaire sont sélectionnés parmi les acides 2-, 3- et 4-aminobenzoïque, 2-, 3- et 4-phénylacétique, 2,3-, 2,4-, 2,5-, 3,4- et 3,5-diaminobenzoïque, 4- ou 5-aminosalicylique, 3-amino-4-hydroxybenzoïque, 4-amino-3-hydroxybenzoïque, 2-, 3- et 4-aminobenzènesulfonique, 4-amino-3-hydroxynaphtalène-1-sulfonique, 3-aminophtalique, 5-amino-isophtalique.

7. Agents permettant de colorer des fibres contenant de la kératine selon les revendications 1 à 3, caractérisés en ce que les dérivés d'aniline de la formule II sont sélectionnés parmi les composés suivants: 1,2,4,5-tétra-aminobenzène, 2,4,5-triaminophénol, pentaminobenzène, hexa-aminobenzène, 2,4,6-triaminorésorcine, 4,5-diamino-pyrocatéchine, 4,6-diaminopyrogallol, 2,5-dihydroxy-4-morpholinoaniline, 3,5-diamino-4-hydroxy-pyrocatéchine.

8. Agents permettant de colorer des fibres contenant de la kératine selon les revendications 1 à 3, caractérisés en ce que les dérivés d'aniline de la formule III sont sélectionnés parmi les composés suivants: 2-(2,5-diaminophényl)-éthanol, 3-aminométhyl-4-aminophénol, 3-(2,5-diaminophényl)-propanol, 2-aminométhyl-4-aminophénol, 2-aminométhyl-5-aminophénol, 2-(2,4-diaminophényl)-éthanol.

9. Agents permettant de colorer des fibres contenant de la kératine selon les revendications 1 à 3, caractérisés en ce que les dianilines de la formule IV sont sélectionnés parmi les composés suivants: 4,4'-diaminostilbène, acide 4,4'-diaminostilbène-2,2'-disulfonique, sel sodique, 4,4'-diaminodiphénylméthane, sulfure de 4,4'-diaminodiphényle, sulfoxyde de 4,4'-diaminodiphényle, 4,4'-diaminodiphénylamine, 4,4'-diaminobenzophénone, 4,4'-diaminobenzodiphényléther, 3,3',4,4'-tétraaminodiphényle, 3,3',4,4'-tétra-aminobenzophénone, 1,3-bis-(2,4-diaminophénoxy)-propane, 1,8-bis-(2,5-diaminophénoxy)-3,6-dioxaoctane.

10. Agents permettant de colorer des fibres contenant de la kératine selon les revendications 1 à 3, caractérisés en ce que les hétérocycles non aromatiques non substitués ou substitués par un des groupes amino-(alkyle en C₁-C₄), hydroxy(alkyle en C₁-C₄) ou carboxyle, sont sélectionnés parmi les composés suivants: pipéridine, pipérazine, 1-(2-aminooctyl)-pipérazine, 1-(2-hydroxyéthyl)-pipérazine, 3-pyrroline, pyrrolidine, thiazolidine, acides thiazolidine-4-carboxylique, pipéridine-2-carboxylique, pipéridine-3-carboxylique, pipéridine-4-carboxylique.

11. Agents permettant de colorer des fibres contenant de la kératine selon les revendications 1 à 3, caractérisés en ce que le hexosamine est sélectionné parmi la D-glucosamine et la D-galactosamine.

12. Agents permettant de colorer des fibres contenant de la kératine selon les revendications 1 à 11, caractérisés en ce qu'ils contiennent au moins un sel sélectionné parmi le groupe des sels d'ammonium, de lithium, sodium, potassium, magnésium, calcium, strontium, baryum, aluminium, titane, manganèse, fer, cobalt, nickel, cuivre, argent, zinc, lanthane, cérium, praséodyme, néodyme et gadolinium.

13. Agents permettant de colorer des fibres contenant de la kératine selon la revendication 12, caractérisés en ce que le sel est sélectionné parmi le groupe constitué du carbonate et de l'acétate d'ammonium, de l'acétate de sodium, de lithium et de potassium, du glycolate et du lactate de sodium, et du gluconate de calcium.

14. Teintures capillaires renfermant au moins une isatine de la formule I, selon la revendication 1, dans une proportion de 0,3 à 65, de préférence de 6 à 20 mmoles, au moins une amine aliphatique primaire, qui porte dans la chaîne C au moins un groupe NR⁸R⁹ ou OR¹⁰ supplémentaire, dans une proportion de 0,3 à 65, de préférence de 6 à 20 mmoles, dans chaque cas pour 100 g de la préparation de teinture capillaire prête à l'emploi, ainsi qu'un vecteur aqueux.

15. Teintures capillaires renfermant au moins une isatine de la formule I, selon la revendication 1, dans une proportion de 0,3 à 65, de préférence de 6 à 20 mmoles, au moins un composé aromatique hétérocyclique ou isocyclique sans groupe amino primaire, dans une proportion de 0,3 à 65, de préférence de 6 à 20 mmoles, dans chaque cas pour 100 g de la préparation de teinture capillaire prête à l'emploi, ainsi qu'un vecteur aqueux.

16. Teintures capillaires renfermant au moins une isatine de la formule I, selon la revendication 1, dans une proportion de 0,3 à 65, de préférence de 6 à 20 mmoles, au moins un acide carboxylique ou sulfonique aromatique comportant un groupe amino primaire, dans une proportion de 0,3 à 65, de préférence de 6 à 20 mmoles, dans chaque cas pour 100 g de la préparation de teinture capillaire prête à l'emploi, ainsi qu'un vecteur aqueux.

17. Teintures capillaires renfermant au moins une isatine de la formule I, selon la revendication 1, dans une proportion de 0,3 à 65, de préférence de 6 à 20 mmoles, au moins un dérivé d'aniline de la formule II, dans une proportion de 0,3 à 65, de préférence de 6 à 20 mmoles, dans chaque cas pour 100 g de la préparation de teinture capillaire prête à l'emploi, ainsi qu'un vecteur aqueux.

18. Teintures capillaires renfermant au moins une isatine de la formule I, selon la revendication 1, dans une proportion de 0,3 à 65, de préférence de 6 à 20 mmoles, au moins un dérivé d'aniline de la formule III, dans une proportion de 0,3 à 65, de préférence de 6 à 20 mmoles, dans chaque cas pour 100 g de la préparation de teinture capillaire prête à l'emploi, ainsi qu'un vecteur aqueux.

19. Teintures capillaires renfermant au moins une isatine de la formule I, selon la revendication 1, dans une proportion de 0,3 à 65, de préférence de 6 à 20 mmoles, au moins un dérivé de dianiline de la formule IV, dans une proportion de 0,3 à 65, de préférence de 6 à 20 mmoles, dans chaque cas pour 100 g de la préparation de teinture capillaire prête à l'emploi, ainsi qu'un vecteur aqueux.

20. Teintures capillaires renfermant au moins une isatine de la formule I, selon la revendication 1, dans une proportion de 0,3 à 65, de préférence de 6 à 20 mmoles, au moins un composé hétérocyclique aromatique non substitué ou substitué par un des groupes amino-(alkyle en C₁-C₄), hydroxy-(alkyle en C₁-C₄) ou carboxyle, dans une proportion de 0,3 à 65, de préférence de 6 à 20 mmoles, dans chaque cas pour 100 g de la préparation de teinture capillaire prête à l'emploi, ainsi qu'un vecteur aqueux.

21. Teintures capllaires renfermant au moins une isatine de la formule I, selon la revendication 1, dans une proportion de 0,3 à 65, de préférence de 6 à 20 mmoles, au moins une hexosamine, dans une proportion de 0,3 à 65, de préférence de 6 à 20 mmoles, dans chaque cas pour 100 g de la préparation de teinture capillaire prête à l'emploi, ainsi qu'un vecteur aqueux.

22. Teintures capillaires selon les revendications 14 à 21, renfermant en outre un sel sélectionné parmi le groupe des sels d'ammonium, de lithium, sodium, potassium, magnésium, calcium, strontium, baryum, aluminium, titane, manganèse, fer, cobalt, nickel, cuivre, argent, zinc, lanthane, cérium, praséodyme, néodyme ou gadolinium, dans une proportion de 0,3 à 65, de préférence de 2 à 15 mmoles, pour 100 g de la préparation de teinture prête à l'emploi.
